# EUROPÄISCHE PATENTSCHRIFT

(11) **EP 1 812 116 B1**
(45) Veröffentlichungstag und Bekanntmachung des Hinweises auf die Patenterteilung: **05.10.2011**
(21) Anmeldenummer: 05738383.8
(22) Anmeldetag: 22.04.2005
(51) Int. Cl.: A61Q 1/12, A61Q 19/00, A61K 8/25, A61Q 15/00

(54) **SCHWEISSABSORBIERENDES KOSMETISCHES PRODUKT UND VERFAHREN ZU SEINER HERSTELLUNG**
PERSPIRATION-ABSORBENT COSMETIC PRODUCT AND METHOD FOR THE PRODUCTION THEREOF
PRODUIT COSMETIQUE ABSORBANT LA SUEUR ET SON PROCEDE DE FABRICATION

(30) Priorität: 22.04.2004 DE 102004020647
(43) Veröffentlichungstag der Anmeldung: 01.08.2007
(73) Patentinhaber: Coty B.V., 2031 CC Haarlem (NL)
(72) Erfinder: HWANG, Donna Hui-Ing, Leonia, New York 07605 (US); MACCHIO, Ralph, Sparta, New York 07971 (US); CERNASOV, Domnica, Ringwood, New York 07456 (US); BARONE, Salvatore, Staten Island, New York 10314 (US)
(74) Vertreter: Reinstädler, Diane
(86) Internationale Anmeldenummer: PCT/EP2005/004496
(87) Internationale Veröffentlichungsnummer: WO 2005/102264

(56) Entgegenhaltungen:
- EP-A- 0 139 481
- EP-A- 0 701 812
- EP-A- 1 338 266
- WO-A-03/026608
- WO-A-03/105790
- DE-A1- 10 208 550
- US-A- 6 025 007
- US-A1- 2003 186 826
- US-A1- 2004 001 799

## Beschreibung

Die Erfindung betrifft ein wasserhaltiges oder wasserfreies schweißabsorbierendes kosmetisches Produkt, beispielsweise ein Antiperspirant, enthaltend eine Basisformulierung sowie einen schweißabsorbierenden Komplex. Die Erfindung betrifft ferner ein Verfahren zur Herstellung des Komplexes und des kosmetischen Produkts.

Kosmetische Produkte mit einer Antischweißwirkung sind bekannt. Es werden unterschiedliche Produktklassen unterschieden. Deodorants eliminieren unangenehme Gerüche, die von der bakteriellen Zersetzung von Schweiß entstehen. Deodorants enthalten daher häufig antibakterielle Substanzen sowie wohlriechende Substanzen, wie Duftstoffe. Viele ätherische Öle weisen sowohl antibakterielle als auch wohlriechende Eigenschaften auf, weswegen sie häufig in Deodorants Verwendung finden. Produkte, welche die Schweißproduktion an sich hemmen - so genannte Antiperspirantien - enthalten als Wirkstoff Adstringentien (Antiperspirantaktiva), insbesondere Aluminium- oder Zirkoniumsalze. Deodorantprodukte sind grundsätzlich wasserhaltig, wohingegen traditionelle Antiperspirantien (die in einer unpolaren Phase, wie Silikonöl oder Mineralöl, suspendierte Adstringentien enthalten) im Wesentlichen wasserfrei sind, um die Polymerisation der Adstringentien zu verhindern. Diese Art von Antiperspirantprodukte liegen gewöhnlich in einer festen Stiftform vor. Dennoch wurde unlängst ein neue Generation transparenter Antiperspirantien entwickelt, die aufgrund ihres ästhetischen Erscheinungsbildes an Popularität gewonnen hat. Diese neue Generation von Antiperspirantien sind Emulsionen, die üblicherweise Propylenglykol, Adstringentien, Wasser und Öle enthalten. Dieser Typus von Antiperspirantien ist jedoch wegen der Polymerisation der Adstringentien weniger aktiv als der traditionelle Typ.

Beide Produktklassen - Deodorants und Antiperspirantien - können zusätzlich Substanzen enthalten, die in der Lage sind, Schweiß zu absorbieren und so ein frisches und trockenes Hautgefühl zu vermitteln. Übliche schweißabsorbierende Komponenten sind beispielsweise Polymere, wie natürliche oder chemisch modifizierte Polysaccharide oder Polysaccharid-Gummis oder synthetische Polymere. Derartige Polymere bilden dreidimensionale Netzwerke aus, die in der Lage sind, Wasser aufzunehmen. Ein gemeinsames Problem von wasserhaltigen Produkten (umfassend Deodorants und Antiperspirantien vom Emulsionstyp) ist, dass die wasserabsorbierende Komponente schnell ihre Absorptionsfähigkeit verliert, sobald sie in eine wasserhaltige Formulierung eingebracht wird. Andererseits zeigen wasserabsorbierende Komponenten in traditionellen wasserfreien Antiperspirantien nur eine geringe Wasser- bzw. Schweißabsorptionsaktivität, da sie von einem unpolaren Medium umgeben sind, wodurch der Kontakt mit dem Wasser/Schweiß der Haut verhindert wird.

Aus WO 03/030853 A ist ein im Wesentlichen wasserfreies Unterarmprodukt bekannt, das ein spezielles wasserabsorbierendes Polymer, flüchtiges Silikon und ein Geilerungsmittel enthält und optional geringe Mengen eines Tensids (zur Stabilisierung der Formulierung), eines Antiperspirantwirkstoffs oder deodorierenden Mittels, nicht-flüchtigen Silikons und eines Emulgators. Das Produkt ist eine Suspension, in der das wasserabsorbierende Polymer in Form von dispergierten Partikeln vorliegt. Diese Formulierung ist nicht geeignet, um in übliche wasserhaltige Formulierungen eingesetzt zu werden, da das wasserabsorbierende Polymer in Wasser quillt und seine Wasserspeicherfähigkeit verliert.

Aufgabe der vorliegenden Erfindung ist; ein schweißabsorbierendes kosmetisches Produkt, insbesondere ein Antiperspirantprodukt, zur Verfügung zu stellen, das eine erhöhte und langanhaltende Wasserabsorptionsfähigkeit insbesondere in weitgehend wasserfreien aber auch in wasserhaltigen (Emulsionstyp) Formulierungen aufweist und die Formulierungsprobleme bekannter Antiperspirantprodukte überwindet.

Diese Aufgabe wird durch ein schweißabsorbierendes kosmetisches Produkt mit den Merkmalen des Anspruchs 1 gelöst. Das erfindungsgemäße Produkt enthält (in Massenantellen):
i) eine Basisformulierung und
ii) einen schweißabsorbierenden Komplex, umfassend
   (a) 0,1 bis 90 Gew.-% mindestens einer wasserabsorbierenden Komponente,
   (b) 0,1 bis 80 Gew.-% mindestens eines oberflächenaktiven Agens und (c) 0 bis 50 Gew.-% mindestens eines Lösungsmittels und/oder mindestens einer Trägersubstanz.

Dabei ist entscheidend, dass der schweißabsorbierende Komplex in der Basisformulierung in Form emulgierter Partikel vorliegt, welche aus einem dreidimensionalen, in Wasser quellbaren Polymernetzwerk der mindestens einen wasserabsorbierenden Komponente bestehen und welche mit dem mindestens einen oberflächenaktiven Agens zumindest teilweise umhüllt (beschichtet) sind.

In dem Komplex ist die mindestens eine wasserabsorbierende Komponente in der Lage, in Wasser zu quellen (ohne jedoch in der Zusammensetzung vollständig gelöst vorzuliegen) und hat eine ausreichende Stärke, im Kontakt mit Wasser ein dreidimensionales gelartiges Netzwerk auszubilden. In diesem Komplex ist die mindestens eine wasserabsorbierende Komponente durch eine Beschichtung (Hülle) aus dem zumindest einen oberflächenaktiven Agens (Tensid) geschützt. Der Komplex ist als Zusatz zu beliebigen Basisformulierungen bestimmt, um ein kosmetisches Produkt zu bereitzustellen. Die umhüllende Schicht des mindestens einen Tensids suspendiert das Absorbermaterial gleichmäßig innerhalb der Basisformulierung und stabilisiert die Absorberpartikel, wodurch ihre Schweißabsorptionsfähigkeit konserviert wird. Die Interaktion zwischen Tensid(en) und wasserabsorbierende(n) Komponente(n) basiert auf Wasserstoffbrückenbindungen, wobei das oberflächenaktive Agens eine orientierte Schicht ausbildet und mit seinen lipophilen Segmenten agiert, um das Absorbermaterial innerhalb der Formulierung zu stabilisieren. Nach Auftragen auf die Haut bildet die den Komplex enthaltende Formulierung einen dünnen Film auf der Haut aus, wobei eine Reorganisation der Tenside stattfindet. Das bedeutet, dass die suspendierenden Tenside durch die Filmbildung sich zwischen der Haut und der wasserabsorbierenden Komponente anordnen und somit den Kontakt zwischen der Haut und der wasserabsorbierenden Komponente vermitteln, der die Schweißabsorption ermöglicht. Dieser Vorgang erlaubt der wasserabsorbierenden Komponente die rasche Absorption vön Schweiß aus der Haut und auch, die Wasserabsorptionskapazität aufrechtzuerhalten.

Obwohl der Komplex in erster Linie entworfen wurden, um weitgehend wasserfreien Antiperspirantbasisformulierungen zugesetzt zu werden, ist er auch für die Verwendung in wasserhaltigen Formulierungen beispielsweise der neuen Generation von Antiperspirantien vom Emulsionstyp geeignet, ohne seine Wasserabsorptionskapazität einzubüßen. In diesem Fall schützt die umhüllende Schicht des mindestens einen oberflächenaktiven Agens das Absorbermaterial vor unerwünschter Wasseraufnahme aus der wässrigen Phase der Formulierung. Auf diese Weise wird nur ein geringer Anteil des vorhandenen Absorbermaterials im Wasser der Formulierung gequollen vorliegen, wodurch der Hauptanteil der Wasserspeicherkapazität für die Schweißaufnahme zur Verfügung steht. Im Ergebnis bleibt die hohe Schweißabsorptionskapazität des Absorbermaterials über eine längere Dauer als bei bekannten Zusammensetzungen_erhalten.

Zusätzlich stellt das Netzwerk der zumindest einen wasserabsorbierenden Komponente ein offenes dreidimensionales Gerüst zur Verfügung, in dessen Hohlräume die hydrophilen Segmente des zumindest einen Tensids oder auch andere hydrophobe Materialien einlagern können und auf diese Weise die Aufnahme und Zurückhaltung von Wasser unterstützen. Die Einlagerung hydrophober Materialien oder Segmente verhindert übermäßig starke Wasserstoffbrückenbindungen der Backbone-Ketten der wasserabsorbierenden Komponente, so dass Wasser/Schweiß leicht eindringen und die Absorbermoleküle auseinander drängen kann, um diese rasch zu hydratisieren. Mit anderen Worten werden die inter- und intramolekularen Wasserstoffbrückenbindungen durch das hydrophile Material getrennt, um den Eintritt von Wasser zu erleichtern. Zu diesem Zweck wird gemäß einer bevorzugten Ausgestaltung der Erfindung eine Mischung aus zwei oder mehreren wasserabsorbierenden Komponenten in dem Komplex eingesetzt, wobei mindestens einer von diesen ein hydrophiles Material darstellt. Typische hydrophile Materialien können diesem Zweck dienen, beispielsweise hydratisierte Kieselsäure (siehe unten).

Der schweißabsorbierende Komplex und das erfindungsgemäße kosmetrische Produkt weisen eine überragende Schweißabsorptionsfähigkeit, Feuchtigkeitsreduzierung, Inhibition von Mikroorganismen, und schließlich einen deutlichen deodorierenden Nutzen auf. Der Komplex ist geeignet, in jegliches kosmetische Produkt eingebracht zu werden, insbesondere in wasserfreie aber ebenso in wasserhaltige Produkte, wie Unterarmantiperspirantien.

Nach besonders vorteilhaften Ausgestaltungen der Erfindung umfasst der schweißabsorbierende Komplex
(a) 10 bis 80 %, insbesondere 20 bis 70 % und bevorzugt etwa 30 bis 50 % mindestens einer wasserabsorbierenden Komponente,
(b) 10 bis 70 %, insbesondere 20 bis 60 % und bevorzugt etwa 30 bis 45 % mindestens eines oberflächenaktiven Agens und
(c) optional 0 bis 50 % mindestens eines Lösungsmittels und/oder mindestens einer Trägersubstanz.

Die Bereiche können abhängig von den Eigenschaften der einzelnen Komponenten sowie der Art des kosmetischen Produkts stark variieren.

Die mindestens eine wasserabsorbierende Komponente ist im Wesentlichen nicht in dem mindestens einen Lösungsmittel und/oder der Trägersubstanz löslich, andererseits aber in der Lage, in Wasser zu quellen. Im Kontakt mit Wasser bildet sie ein dreidimensionales gelartiges Polymernetzwerk aus, resultierend in Partikel, die nach wie vor zumindest bereichsweise mit dem oberflächenaktiven Agens beschichtet sind. Die wasserabsorbierende Komponente weist eine Wasserabsorptionskapazität von mindestens 20 %, insbesondere mindestens 50 % bezogen auf ihre Trockenmasse auf. Üblicherweise liegt ihre Wasserabsorptionskapazität zwischen 20 und 300 % der Trockenmasse. Die mittlere Partikelgröße der wasserabsorbierenden Komponente im Komplex liegt im Bereich von 0,1 bis 450 µm, insbesondere von 10 bis 200 µm. Partikel mit mittleren Partikelgrößen zwischen 10 bis 60 µm zeigen besonders gute Ergebnisse.

Das kosmetische Produkt, welches den oben beschriebenen schweißabsorbierenden Komplex enthält, kann aus einer vielfältigen Produktgruppe gewählt sein. Diese Produktgruppe umfasst Antiperspirantien und Deodorants (insbesondere Unterarmprodukte), jegliche adstringierende und/oder deodorierende Präparationen, Stifte, Sprays, Aerosole, Cremes, Sonnenschutzmittel, Aftershaves, Lotionen, Grundierungscremes und Make-ups. Ein Vorteil des erfindungsgemäßen Komplexes ist, dass er ohne weiteres zu beliebigen Standardzubereitungen (Basisformulierungen) zugesetzt werden kann, ohne seine Wasserspeicherfähigkeit zu verlieren. Solche Basisformulierungen umfassen insbesondere wasserfreie Antiperspirantformulierungen, die mindestens ein in der unpolaren Phase suspendiertes Adstringent enthalten, als auch wasserhaltige Adstringentformullerungen des Emulsionstyps der neuen Generation. Eine typische wasserfreie Antiperspirantbasis enthält 30 bis 70 %, insbesondere 50 bis 60 Gew.-% mindestens eines unpolaren Lösungsmittels (beispielsweise Silikonöl) ; 10 bis 30 %, insbesondere etwa 20 Gew.-% mindestens eines Gelierungsmittels/Verdickungsmittels (beispielsweise Stearylalkohol); 10 bis 30 %, insbesondere etwa 20 Gew.-% mindestens eines Adstringenzes (beispielsweise Aluminium- und/oder Zirkoniumverbindungen) sowie 5 bis 20 %, insbesondere etwa 10 Gew.-% eines Wachses (zur Herstellung eines Stiftproduktes).

Der schweißabsorbierende Komplex kann grundsätzlich in einem weiten Konzentrationsbereich von 0,05 bis 99 Gew.-% in kosmetischen Produkten eingesetzt werden. Üblicherweise ist der Komplex in der Basisformulierung im Bereich von 0,1 bis 10 Gew.-%, vorzugsweise von 1 bis 6 % enthalten.

Das kosmetische Produkt kann selbstverständlich weitere Hilfsstoffe und/oder Wirkstoffe (aktive Agenzien) enthalten, beispielsweise Pigmente, Farbstoffe, Antioxidantien, Konservierungsmittel, andere feuchtigkeitsspeichemde Substanzen, Weichmacher, Duftstoffe, Stabilisatoren, Adstringentien, Zellturnover-Promotoren, Zellproliferationsstimulatoren, entzündungshemmende Agenzien, antimikrobielle Agenzien, Hormonregulatoren, Enzyminhibitoren, UV-Absorber, Sonnenschutzstoffe und dergleichen sowie Mischungen von diesen.

Wie bereits erwähnt, zeichnet sich der erfindungsgemäße schweißabsorbierende Komplex durch eine Beschichtung/Überzug der Absorberpartikel mit dem mindestens einen Tensid aus. Dieses Gefüge kann in einfacher Weise erhalten werden, indem die mindestens eine wasserabsorbierende Komponente und das mindestens eine oberflächenaktive Agens unter Rühren und Wärmezufuhr miteinander vermischt werden, bis eine homogene Mischung erhalten wird. Die Zufuhr von thermischer Energie (Wärme) und Scherkräften (durch Rühren) in diesem Schritt ist entscheidend, um inter- und intramolekulare Bindungen innerhalb des oberflächenaktiven Agens und der wasserabsorbierenden Komponente aufzubrechen und im anschließenden Abkühlungsprozess die Bildung neuer physikalischer Wechselwirkungen (insbesondere hydrophobe Wechselwirkungen und Wasserstoffbrückenbindungen) zwischen dem zumindest einen Tensid und der zumindest einen wasserabsorbierenden Komponente zu ermöglichen. Ohne diese Zuführung von Wärme und Scherkräften bilden Tensid(e) und wasserabsorbierende Komponente(n) nur sehr geringe physikalische Wechselwirkungen aus und vermögen einem kosmetischen Produkt - nach Zusetzung zu einer kosmetischen Basisformulierung - keine signifikante Wasserabsorptionskapazität zu verleihen. Falls ein Lösungsmittel und/oder eine Trägersubstanz verwendet wird, wird dieses zu der Mischung aus wasserabsorbierender Komponente und oberflächenaktivem Agens zugegeben und bei der angehobenen Temperatur weiter gerührt, bis eine im Wesentlichen homogene Mischung vorliegt.

Um das kosmetische Produkt herzustellen, wird der so vorgefertigte schweißabsorbierende Komplex, der bereits die umhüllten Absorberpartikel enthält, einfach einer geeigneten Basisformulierung zugesetzt, insbesondere einer Antiperspitantbasisformulierung.

Nachfolgend werden geeignete Substanzen für die einzelnen Inhaltsstoffe des erfindungsgemäßen schweißabsorbierenden Komplexes genannt.

### Wasserabsorbierende Komponente

Die mindestens eine wasserabsorbierende Komponente wird ausgewählt aus beliebigen natürlichen oder synthetischen Polymeren, die in Wasser zu quellen vermögen und dabei ein dreidimensionales gel-artiges Netzwerk ausbilden.

Die vorliegend meist bevorzugten wasserabsorbierenden Komponenten stellen Gummis beziehungsweise gummiartige Polymere dar. Im Rahmen der vorliegenden Erfindung wird "Gummi" allgemein als ein beliebiges wasserlösliches Polymer definiert, das aus Land- oder Meerespflanzen oder -mikroorganismen isoliert werden kann und das die Fähigkeit besitzt, zur Viskosität und/oder Quellfähigkeit seiner Dispersion beizutragen. Die wasserabsorbierende Komponente ist vorzugsweise ein aus pflanzlicher oder mikrobieller Biosynthese, von Land oder Meeresorganismen stammender Gummi. Solche Gummis enthalten im Wesentlichen Wiederholungen von Monosaccharideinheiten and weisen relativ hohe Molmassen von vorzugsweise mindestens 100.000 g/mol auf. Beispiele bevorzugter Gummis sind Guar-Gummi (Cyamopsis tetragonolobus-Gummi), Guar-Derivate, Johannisbrotkernmehl, Scleroglucan (Sclerotium gum), Tamarindenkernmehl, Dextrin-Gummi und dergleichen sowie Derivate und Mischungen von diesen.

Eine weitere bevorzugte Klasse Wasserabsorbierender Komponenten stellen Polysaccharide pflanzlichen Ursprungs dar, die im Wesentlichen eine Wiederholung von Monosaccharideinheiten von Hexosen und/oder Pentosen umfassen. Besondere Beispiele umfassen natürliche Cellulosen, natürliche Fasern, Cellulose-Derivate, mikrokristalline Cellulose, Methylcellulosen, Methoxycellulosen, Hydroxyethylcellulosen, Hydroxypropylcellulosen, Hydroxypropylmethylcellulosen, Dextrine, Maltodextrine, Inulin, Inulin-Derivate, Stärke, Stärke-Derivate, Derivate und Mischungen von diesen.

Noch eine andere Gruppe geeigneter wasserabsorbierender Komponenten wird aus chemisch modifizierten-Polysacchariden gewählt, insbesondere aus der Gruppe enthaltend Cellulose-Derivate, Stärke-Derivate, Pektin-Derivate, Stärkepfropfpolymere (Stärke/Acrylamid/Natriumacrylat-Pfropfcopolymere) und Mischungen von diesen.

Gemäß einer weiteren Ausgestaltung der Erfindung wird die mindestens eine wasserabsorbierende Komponente aus Polyacrylat-basierten synthetischen Polymeren gewählt, die insbesondere Wiederholungen von Acrylsäure, Acrylamid, Methacrylsäure umfassen, einschließlich Derivate, Copolymere oder Mischungen von diesen. Polyacrylamide stellen ein besonderes Beispiel für diese Verbindungen dar.

Eine andere Gruppe bevorzugter wasserabsorbierender Komponenten ist gewählt aus Kieselsäuren (Si(OH)₄ bzw. SiO₂) und beliebigen Arten ihrer Derivate und Modifikationen.

Geeignete Beispiele umfassen ihre Kondensationsprodukte Polykieselsäuren ((SiO₂)ₘ x nH₂O), Kieselsäureanhydrid (Silica, SiO₂), pyrogene Kieselsäure ("Fumed Silica"), hydratisierte Kieselsäure (SiO₂ x nH₂O), Silicagel, und/oder Silikatester. Es wurde gefunden, dass die Wasserabsorptionsrate und -kapazität dieser Kieselsäurenkomponenten dramatisch verstärkt werden kann, indem sie vor der Herstellung des Komplexes in einer Ölphase zunächst vordispergiert werden.

Selbstverständlich können Mischungen der vorstehenden wasserabsorbierenden Komponenten ebenfalls eingesetzt werden. Es ist besonders bevorzugt vorgesehen, eine Mischung aus einer Komponente der Kieselsäuregruppe und einer Komponente.der anderen Gruppen als wasserabsorbierende Komponente des erfindungsgemäßen Komplexes einzusetzen.

### Oberflächenaktive Agenzien

Wie bereits ausgeführt, hat das mindestens eine oberflächenaktive Agens die Funktion, eine Beschichtung auf der äußeren Oberfläche der Partikel der wasserabsorbierenden Komponente(n) auszubilden, wodurch ihre Oberflächeneigenschaften beeinflusst werden. Insbesondere helfen die oberflächenaktiven Agenzien, das Polymer auf der Haut zu halten, stabilisieren die Partikel in der Formulierung und erleichtern die Einlagerung von Wasser. Oberflächenaktive Agenzien umfassen Verbindungen - enthaltend Monomere, Dimere, Trimere, Oligomere und Polymere -, die lipophile genauso wie hydrophile Funktionalitäten ausreichender Stärke aufweisen, um Affinitäten sowohl gegenüber hydrophilen als auch gegenüber lipophilen Anteilen der Formulierung auszubilden. Solche Agenzien bilden orientierte Schichten um die wasserabsorbierenden Partikel aus. Auf diese Weise werden die Partikel der wasserabsorbierenden Komponente(n) innerhalb der Formulierung stabilisiert und homogen verteilt und gegebenenfalls vor unerwünschter Wasserabsorption aus einer wasserhaltigen Basisformulierung bewahrt.

Für die oben genannten Zwecke weist das oberflächenaktive Agens eine so genannte Hydrophilie/Lipophilie-Balance (HLB) von mindestens 4,5, insbesondere mindestens 7 auf, vorzugsweise im Bereich von 12 bis 18. Sehr gute Resultate werden mit Tensiden mit einem HLB im Bereich von 14 bis 16 erhalten. Die genannten HLB-Werte werden vorwiegend dann gewählt, wenn der Komplex zur Verwendung in wasserfreien Formulierungen bestimmt ist, beispielsweise in traditionellen Antiperspirantien. Wenn andererseits der Komplex für den Zusatz zu wasserhaltigen Formulierungen vorgesehen ist, beispielsweise Antiperspirantien vom Emulsionstyp, werden niedrigere HLB-Werte bevorzugt. Im letztgenannten Fall wird ein HLB unterhalb von 13, insbesondere im Bereich von 2 bis 13, vorzugsweise von 3 bis 11 gewählt. Tensiden mit einem HLB im Bereich von 5 bis 10 sind für wasserhaltige Anwendungen besonders bevorzugt. Der HLB ist ein von C. Griffin entwickelter dimensionsloser Wert, der die relativen Anteile der lipophilen gegenüber der hydrophilen Segmente eines Materials berücksichtigt. Die Zuweisung von numerischen Werten für den HLB basiert auf Effekten der chemischen Gruppen innerhalb des Moleküls (D.L. Courtney, in "Surfactants in Cosmetics", 2. Auflage, Marcel Dekker Inc., New York, 1997, 128-130).

Gemäß einer bevorzugten Ausgestaltung der Erfindung wird eine Mischung von mindestens zwei oberflächenaktiven Agenzien in dem Komplex verwendet. In diesem Fall können Tenside mit HLB-Werten auch außerhalb der oben angegebenen Bereiche eingesetzt werden, vorausgesetzt, dass ein effektiver gewichteter mittlerer HLB der Kombination in die oben genannten Bereiche fällt. Auch hier wird der HLB in Abhängigkeit von der beabsichtigten Verwendung des Komplexes gewählt, das heißt in wasserfreien oder wasserhaltigen Formulierungen.

Das mindestens eine oberflächenaktive Agens kann eine nicht-ionische, kationische oder amphotere Verbindung oder einen Kombination von solchen sein. Ausgewählte Beispiele enthalten Fettalkohole, ethoxylierte Alkohole, ethoxylierte Triglyceride, ethoxylierte Öle, Monoglyceride, Carboxylsäureester von Alkyl- oder Alkenylglycolen, C1-C40-Fettsäureester von Polyolen, C1-C40-Fettsäureether von Polyolen, C1-C40- Fettsäureester von Alkyl- oder Alkenylglycolen, Polyglycerinester, Polyglycerinester von C1-C40-Fettsäuren, Kohlenwasserstoff-abgeleitete Ester, Zuckerester und -polyester, alkoxylierte Zuckerester und -polyester, ethoxylierte Carboxylsäureester von C1-C40-Fettsäuren, Sorbitan- oder Polysorbatester von Fettsäuren, ethoxylierte Sorbitanester von Fettsäuren, ethoxylierte Zuckerether von Fettsäuren, alkoxylierte Derivate von C1-C40-Fettsäureestern von C1-C40-Fettalkoholen, alkoxylierte Derivate von C1-C40-Fettsäureethern von C1-C40-Fettalkoholen, Polyethylenglycolether, Polyethylenglycolester, ethoxylierte Polysiloxane, Alkylglycoside, Alkanolamide, Aminoxide, Fettsäureamide, Alkylamidoalkylamine, Alkylamine, Alkylimidazoline, alkylsubstituierte Aminosäuren und Mischungen von diesen. Besondere Beispiele sind Saccharosestearat und Sorbitansesquioleat und Mischungen von diesen.

### Lösungsmittel/Trägersubstanz

Die Gegenwart eines Lösungsmittels und/oder einer Trägersubstanz im schweißabsorbierenden Komplex ist optional. Sie ist in der Regel nur dann notwendig, wenn das mindestens eine oberflächenaktive Agens ein Feststoff ist. Im Falle eines oberflächenaktiven Agens, das als Flüssigkeit vorliegt, besteht keine Notwendigkeit für ein Lösungsmittel und/oder eine Trägersubstanz.

Das mindestens eine Lösungsmittel und/oder die mindestens eine Trägersubstanz kann ausgewählt werden aus der Gruppe enthaltend Glycole, Glycerin, polare und unpolare Öle, Kohlenwasserstoffe, Ether, Ester, mittel- und langkettige Alkohole, alkoxylierte Alkohole, polyhydrierte Alkohole, Polyole und Mischungen von diesen. Besondere Beispiele umfassen Propylenglycol, Dipropylenglycol, Ethylenglycol, Glycerin, Diglycerin, Diacetin, Triacetin, Isopropylpalmitat, Isododecan, Isohexadecan, hydrogeniertes Polydecen, Triglyceride, Mineralöl und Mischungen von diesen.

Weitere bevorzugte Ausgestaltungen-der Erfindung ergeben sich aus den übrigen, in den Unteransprüchen genannten Merkmalen.

Die Erfindung wird nachfolgend in Ausführungsbeispielen anhand der zugehörigen Zeichnungen näher erläutert. Es zeigen:
- Figur 1: zeitlicher Verlauf der Wasserabsorption verschiedener unverarbeiteter wasserabsorbierender Komponenten;
- Figur 2: Wasserspeicherkapazität von unverarbeiteten wasserabsorbierenden Kompo- nenten in einer Antiperspirantbasisformulierung;
- Figur 3: Wasserspeicherkapazität von schweißabsorbierenden Komplexen in einer Antiperspirantbasisformulierung gemäß einer ersten Ausgestaltung der Erfindung,
- Figur 4: Wasserspeicherkapazität von schweißabsorbierenden Komplexen in einer Antiperspirantbasisformulierung gemäß einer zweiten Ausgestaltung der Erfindung und
- Figur 5: Wasserspeicherkapazität von schweißabsorbierenden Komplexen in einer Antiperspirantbasisformulierung gemäß einer dritten Ausgestaltung der Erfindung.

### (1) Herstellung des schweißabsorbierenden Komplexes

Mindestens eine wasserabsorbierende Komponente wird in einen sauberen, trockenen Edelstahlkessel gegeben, der mit einem Rührer ausgestattet ist. Vorzugsweise wird eine vorgemischte Kombination von Kieselsäure oder einem Kieselsäurederivat und einer zweiten wasserabsorbierenden Komponente eingesetzt. Unter langsamen Rühren wird das Gemenge auf eine Temperatur zwischen 50 und 100 °C aufgeheizt und bei dieser Temperatur gehalten. Anschließend wird mindestens ein oberflächenaktives Agens langsam in den Kessel zugegeben. Unter Beibehaltung der Batch-Temperatur von 50-100 °C wird das Gemisch für mindestens weitere 15 Minuten kontinuierlich gerührt, bis ein im Wesentlichen einheitliches (homogenes) Gemisch erhalten wird, das keine ungelösten Rohmaterialien enthält. Für den Fall, dass ein das oberflächenaktive Agens eine Feststoff ist, wird ein Lösungsmittel oder eine Trägersubstanz zu der Mischung gegeben und so lange weitergerührt, bis ein homogene Mischung ohne sichtbare Rohstoffe vorliegt. Abhängig von den einzelnen Inhaltsstoffen hat der so hergestellte Komplex die Konsistenz einer Paste, eines weichen Feststoffes oder eines harten Wachses.

Die genauen Mengen der Inhaltsstoffe hängen von den ausgewählten Substanzen ab. Das Massenverhältnis der wasserabsorbierenden Komponente(n) zu der/dem oberflächenaktive/n Agens/Agenzien beträgt 1 : (0,25-2), vorzugsweise 1 : (0,5-1,5). Die bevorzugte Batch-Temperatur für das Verfahren hängt von den Schmelzpunkten der Inhaltsstoffe, insbesondere der wasserabsorbierenden Komponente und des oberflächenaktive Agens ab. Es variiert zwischen 50 und 100 °C, insbesondere zwischen 60 und 90 °C. Für die meisten Inhaltsstoffe ist eine Batch-Temperatur zwischen 70 und 80 °C geeignet.

### (2) Herstellung eines Antiperspirantstifts

Der nach der in (1) beschriebenen Prozedur hergestellte schweißabsorbierende Komplex wird einer geschmolzenen (wasserfreien) Antiperspirantbasisformulierung unter ständigem Rühren bei einer Batch-Temperatur von 60-90 °C, insbesondere von 70-80 °C, zugesetzt, so dass eine homogene Mischung erhalten wird, die 0,1-10%, insbesondere 1-6 Gew.-% des Komplexes enthält. Die Antiperspirantbasisformulierung enthält üblicherweise 30-70 %, insbesondere 50-60 Gew.-% mindestens eines unpolaren Lösungsmittels (beispielsweise Silikonfluid); 10-30 %, insbesondere etwa 20 % Stearylalkohol; 10-30 %, insbesondere etwa 20 % mindestens eines Adstringenzes (beispielsweise Aluminiumoxidsalz) und 5-20 %, insbesondere etwa 10 % Wachs (um einen Stift zu formen). Die Mischung wird anschließend auf Raumtemperatur heruntergekühlt, um eine Stiftform zu erhalten.

### (3) Wasserabsorptionsverhalten verschiedener wasserabsorbierender Komponenten (unverarbeitete Rohstoffe)

Eine definierte Menge jedes Rohstoffs (unverarbeitete wasserabsorbierende Komponente) wurde in eine Feuchtigkeitskammer mit einer relativen Luftfeuchte von 95-99 % gegeben und dort für acht Wochen inkubiert. Die prozentuale Wasserabsorption jeder Probe wurde durch Differenzwägung der Probe vor und nach der Inkubation in der Feuchtigkeitskammer und durch Division des Massenunterschiedes durch die Probenmasse vor der Inkubation berechnet.

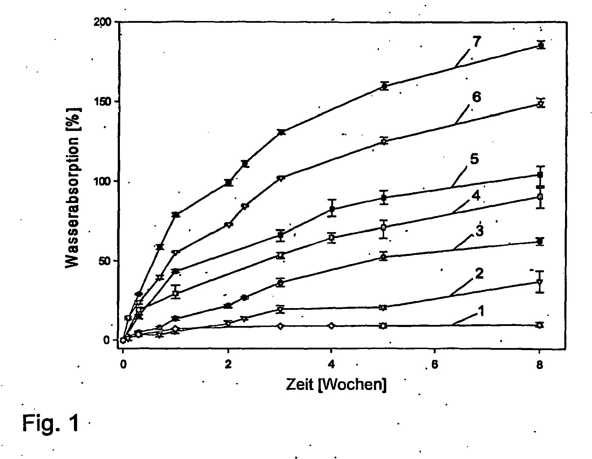

Figur 1 zeigt den achtwöchigen Verlauf der Wasserabsorption einiger Beispiele von potentiellen Kandidaten für die wasserabsorbierende Komponente. Die Ergebnisse folgender wasserabsorbierenden Komponenten sind dargestellt: Mikrokristalline Cellulose (Kurve 1), Kieselsäureanhydrid (Silica) (2), Cellulosepulver (3), Polysaccharid-Gummi (4), Cellulose-Gummi (5), Stärkepfropfpolymer (6) und Salz einer Polyacrylsäure = Natriumpolyacrylat (7). Die Daten zeigen deutlich, dass Polysaccharid-Gummi, Cellulose-Gummi, Stärkepfropfpolymer und Natriumpolyacrylat (Kurven 4-7) jeweils eine Wasserspeicherkapazität von über 100 % bezogen auf die Trockenmasse des Rohmaterials aufweisen. Diese Komponenten werden daher bevorzugt in den erfindungsgemäßen schweißabsorbierenden Komplexen eingesetzt.

### (4) Wasserabsorptionsverhalten verschiedener wasserabsorbierender Komponenten in einer Antiperspirantbasisformulierung (Antiperspirantstift)

Jeweils 5 g der unverarbeiteten wasserabsorbierenden Komponente (Rohmaterial) wurden mit 95 g geschmolzener Antiperspirantbasisformulierung (50-60 Gew.-% Silikonfluid (Cyclomethicon), 18-22 % Stearylalkohol, 20 % Aluminiumoxidsalze, 10 % Wachs und 0,1 % Allantoin) sowie 1,5 g eines Duftstoffs verrührt, bis ein homogenes Gemisch erhalten wurde. Die Mischung wurde auf Raumtemperatur gekühlt, um eine Stickform zu erhalten.

Jeweils eine definierte Menge dieser Mischungen wurde auf Glaswolle in einen geschlossenen Behälter mit Wasserüberschuss platziert und anschließend bei 37 °C für 24 Stunden inkubiert. Eine Probe der reinen, aber duftstoffhaltigen Antiperspirantbasisformullerung wurde auf die gleiche Weise in dem Behälter inkubiert. Nachdem am Ende der Inkubationszeit der Überschuss an Wasser vorsichtig aus dem Behälter dekantiert wurde, wurde die prozentuale Wasserabsorption durch Differenzwägung jeder Probe vor und nach der Wasseraussetzung bestimmt.

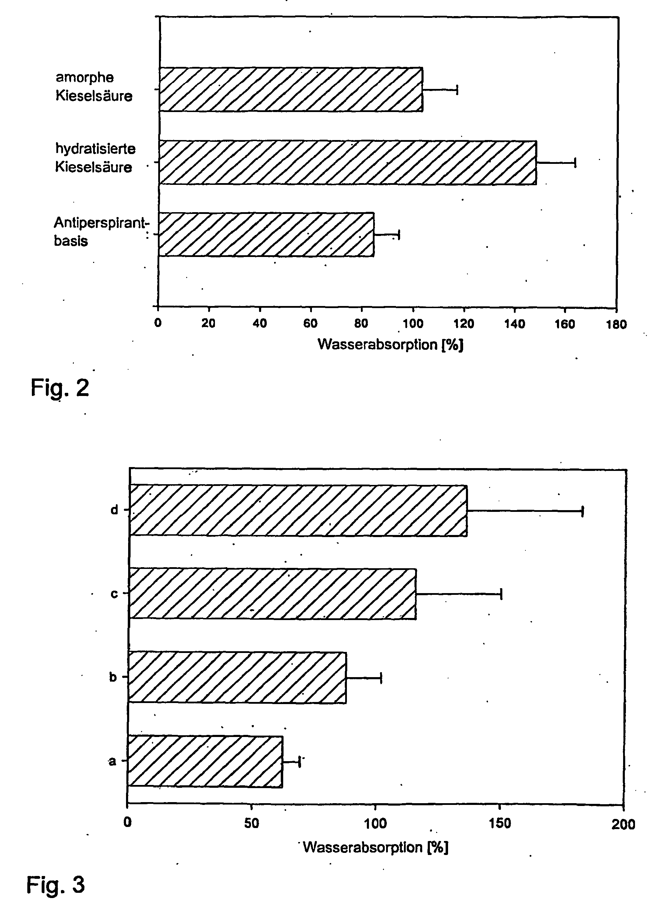

Die Ergebnisse für amorphe Kieselsäure und hydratisierte Kieselsäure sind in Figur 2 dargestellt. Zudem ist die Wasserabsorption der puren Antiperspirantbasisformulierung als Referenz gezeigt. Die Ergebnisse sind in g absorbierten Wassers pro g der "trockenen" Antiperspirantbasis mit bzw. ohne Kieselsäure vor der Inkubation angegeben. Die Daten zeigen, dass die Silica-enthaltenden Antiperspirantien über 50 % mehr Wasser absorbieren als das reguläre Antiperspirant ohne Kieselsäurezusatz. Das belegt, dass der Zusatz von 1 Gew.-% an Kieselsäure einer regulären, wasserhaltigen Antiperspirantbasisformullerung eine zusätzliche Wasserspeicherkapazität von etwa 10 % verleiht.

### (5) Wasserabsorptionsverhalten verschiedener schweißabsorbierender Komplexe in einer Antiperspirantbasisformulierung (Antiperspirantstift)

Ein schweißabsorbierender Komplex gemäß der vorliegenden Erfindung (Komplex A) wurde nach der oben beschriebenen Prozedur (1) hergestellt, wobei eine Mischung aus pyrogener Kieselsäure ("Fumed Silica") und Scleroglucan als wasserabsorbierende Komponente, Saccharoseester als oberflächenaktives Agens und hydrogenisiertes Polydecen als Lösungsmittel eingesetzt wurden. Die Zusammensetzung von Komplex A ist in Tabelle 1 angegeben.

**Tabelle 1:**

| | Komplex A |
|---|---|
| pyrogene Kieselsäure (HDK^{®} N20, Wacker Chemical Corp.) | 3 % |
| Scleroglucan (Alban Muller Ind.) | 30 % |
| Saccharoseester (MMP, Inc.) | 40 % |
| hydrogenisiertes Polydece (Lipo Chemicals, Inc.)n | 27 % |
| **Summe** | 100 % |

Jeweils 2 g und 4 g des Komplexes A wurden mit 96,5 g bzw. 94,5 g einer geschmolzenen Antiperspirantbasisformulierung (50-60 Gew.-% Silikonfluid (Cyclomethicon), 18-22 % Stearylalkohol, 20 % Aluminiumoxidsalze, 10 % Wachs) und mit 1,5 g Duftstoff verrührt, bis ein homogenes Gemisch erhalten wurde. Die Gemische wurden auf Raumtemperatur gekühlt, um eine Stiftform zu erhalten. Die so erhaltenen Produkte enthielten somit 2 bzw. 4 Gew.-% des jeweiligen Gesamtkomplexes und 0,66 bzw. 1,32 Gew.-% der wasserabsorbierenden Komponenten (Kieselsäure + Gummi).

Für Vergleichszwecke wurde eine Mischung hergestellt, indem die einzelnen Inhaltsstoffe des Komplexes A (wie in Tabelle 1 aufgerührt) nacheinander (ohne Vormischung des Komplexes) der geschmolzenen duftstoffhaltigen Antiperspirantbasis zugemischt wurden. Die einzelnen Mengen der Inhaltsstoffe wurden entsprechend eines Gesamtmassenanteils des Komplexes im Produkt von 2 % gewählt.

Jeweils eine definierte Menge dieser Mischungen wurde auf Glaswolle in einen geschlossenen Behälter mit Wasserüberschuss platziert und anschließend bei 37 °C für 24 Stunden inkubiert. In gleicher Weise wurde als Kontrolle die pure duftstoffhaltige Antiperspirantbasis in dem Behälter inkubiert. Nachdem der Überschuss an Wasser vorsichtig aus dem Behälter dekantiert wurde, wurde die prozentuale Wasserabsorption durch Differenzwägung jeder Probe vor und nach der Wasseraussetzung bestimmt.

Die Ergebnisse sind in Figur 3 dargestellt. Balken a repräsentiert die Kontrolle (Antiperspirantbasis). Balken b repräsentiert den Vergleichstest, bei dem die Inhaltsstoffe einzein der Basis zugemischt wurden (ohne vorherige Herstellung des Komplexes). Balken c und d zeigen die Präparationen mit 2 bzw. 4 Gew.-% des vorgemischten Komplexes A in der Antiperspirantbasis. Die Daten zeigen, dass die Antiperspirantien, die 2 bzw. 4 % des Komplexes A enthalten, eine um 87 bzw. 118 % erhöhte Wasserabsorption gegenüber der regulären Antiperspirantbasis aufweisen. Die gleichen Inhaltsstoffe - nach individueller Zumischung - verstärken die Wasserabsorption nur um 41 % verglichen mit der regulären Antiperspirantbasis. Dieses Beispiel belegt deutlich, dass der Komplex in seiner erfindungsgemäßen Konformation essentiell ist, um einer Basisformulierung eine deutlich höhere Wasserspeicherkapazität zu vermitteln als die gleichen, aber individuell zugesetzten Inhaltsstoffe.

### (6) Wasserabsorptionsverhalten verschiedener schweißabsorbierender Komplexe in einer Antiperspirantbasisformulierung (Antiperspirantstift)

Ein schweißabsorbierender Komplex gemäß der vorliegenden Erfindung (Komplex B) wurde nach der oben beschriebenen Prozedur (1) hergestellt, wobei eine Mischung aus pyrogener Kieselsäure ("Fumed Silica") und Maltodextrin als wasserabsorbierende Komponente, Saccharoseester als oberflächenaktives Agens und Cyclomethicon als Lösungsmittel eingesetzt wurden. Die Zusammensetzung von Komplex B ist in Tabelle 2 angegeben.

**Tabelle 2:**

| | Komplex B |
|---|---|
| pyrogene Kieselsäure (HDK^{®} N20, Wacker Chemical Corp.) | 12,5 % |
| Maltodextrin (Grain Processing, Inc.) | 25 % |
| Saccharoseester (MMP, Inc.) | 12,5% |
| Cyclomethicon (Lipo Chemicals, Inc.)n , | 50 % |
| **Summe** | 100 % |

4 g des vorgemischten Komplexes B wurden mit 94,5 g einer geschmolzenen Antiperspirantbasisförmulierung (50-60 Gew.-% Silikonfluid (Cyclomethicon), 18-22 % Stearylalkohol,
20 % Aluminiumoxidsalze, 10 % Wachs, 0,1 % Allantoin) und mit 1,5 g Duftstoff verrührt, bis ein homogenes Gemisch erhalten wurde. Das Gemisch wurde auf Raumtemperatur gekühlt, um eine Stiftform zu erhalten. Das so erhaltene Produkt enthielt somit 4 Gew.-% des Gesamtkomplexes bzw. 1,5 Gew.-% der wasserabsorbierenden Komponenten (Kieselsäure + Maltodextrin).

Eine definierte Menge diese Mischung wurde auf Glaswolle in einen geschlossenen Behälter mit Wasserüberschuss platziert und anschließend bei 37 °C für 24 Stunden inkubiert. In gleicher Weise wurde als Kontrolle eine Probe der puren duftstoffhaltigen Antiperspirantbasis in dem Behälter inkubiert. Nachdem der Überschuss an Wasser vorsichtig aus dem Behälter dekantiert wurde, wurde die prozentuale Wasserabsorption durch Differenzwägung jeder Probe vor und nach der Wasseraussetzung bestimmt.

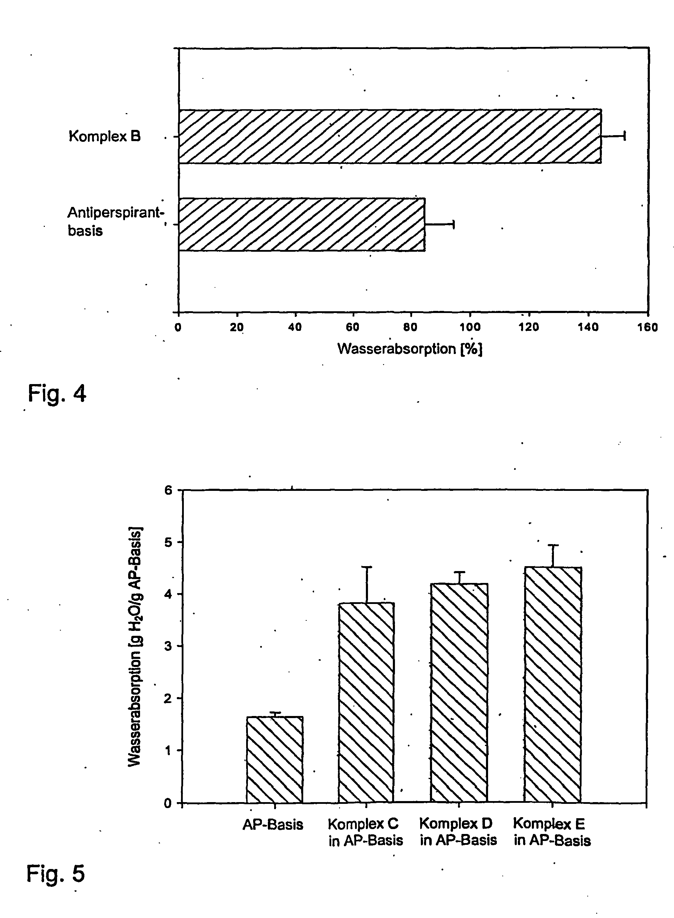

Das Ergebnis ist in Figur 4 dargestellt. Die Daten belegen, dass die Antiperspirantbasisformulierung mit 4 % des Komplexes B die Wasserabsorption um mehr als 70 % verglichen mit der regulären Antiperspirantbasis erhöht.

### (7)_Wasserabsorptionsverhalten verschiedener schweißabsorbierender Komplexe in einer Antiperspirantbasisformullerung (Antiperspirantspray)

Schweißabsorbierende Komplexe C, D und E gemäß der vorliegenden Erfindung wurden nach der oben beschriebenen Prozedur (1) mit den in Tabelle 3 in g oder Gew.-% angegeben Zusammensetzungen hergestellt. Die Komplexe unterscheiden sich im Wesentlichen durch die oberflächenaktiven Agenzien (Sorbitanstearat, Sorbityllaurat, Saccharoseester) und dadurch bedingt in ihren HLB-Werten.

Die schweißabsorbierenden Komplexe C, D und E wurden in ein Antiperspirant-Spraykonzentrat als Antiperspirant-Basisformulierung (AP-Basis) eingearbeitet, so dass homogene Konzentrate für Antiperspirantsprays mit jeweils 20 Gew.-% schweißabsorbierendem Komplex erhalten wurden.

Eine bestimmte Menge jeder Probe wurde auf Glaswolle in einen geschlossenen Behälter eingewogen. Anschließend wurde eine Überschuss an Wasser in den Behälter gegeben und die Proben bei 37 °C für 24 Stunden inkubiert. Als Vergleichsbeispiel wurde ein Antiperspirant-Spraykonzentrat ohne schweißabsorbierendem Komplex analog behandelt. Nach vorsichtigem Dekantieren des überschüssigen Wassers wurden die Proben erneut ausgewogen und die Wasserabsorption durch Differenzwägung vor und nach der Wasseraussetzung bestimmt. Die jeweils auf ein Gramm AP-Basis bezogenen Mengen absorbierten Wassers sind in Figur 5 gezeigt. Die Daten belegen, dass das die Antiperspirant-Sprayprodukte, die Komplex C, D oder E enthalten, eine um 133 %, 155 beziehungsweise 175 % gegenüber der reinen AP-Basis gesteigerte Wasserabsorption aufweisen.

**Tabelle 3**

| | **Komplex C** | **Komplex D** | **Komplex E** |
|---|---|---|---|
| Kieselsäureanhydrid (Silica) | 2,5 | 2,5 | 2,5 |
| (Wacker Chemical Corp.) | | | |
| Taragummi (TIC Gums) | 37,5 | 37,5 | 37,5 |
| Natur-Baumwolle | 0,2 | 0,2 | 0,2 |
| Saccharoseester (MMP, Inc.), HLB-15 | 5 | 15 | - |
| Sorbitanstearat und Sorbityllaurat (HLB ~6) | 30 | 15 | 30 |
| Hydrogeniertes Polydecen (Lipo Chemicals, | 24,8 | 29,8 | 29,8 |
| Inc.) | | | |
| Summe | 100 | 100 | 100 |

## Patentansprüche

1. Schweißabsorbierendes kosmetisches Produkt enthaltend
i) eine Basisformulierung und
ii) einen schweißabsorbierenden Komplex, umfassend
(a) 0,1 bis 90 Gew.-% mindestens einer wasserabsorbierenden-Komponente,
(b) 0,1 bis 80 Gew.-% mindestens eines oberflächenaktiven Agens und
(c) 0 bis 50 Gew.-% mindestens eines Lösungsmittels und/oder mindestens einer
Trägersubstanz,
worin der schweißabsorbierende Komplex Partikel aus einem dreidimensionalen, in Wasser quellbaren Netzwerk der mindestens einen wasserabsorbierenden Komponente ausbildet, welche mit dem mindestens einen oberflächenaktiven Agens zumindest teilweise umhüllt sind und in der Basisformullerung emulgiert sind.

2. Kosmetisches Produkt nach Anspruch 1, wobei der schweißabsorbierenden Komplex
(a) 10 bis 80 Gew.-% der mindestens einer wasserabsorbierenden Komponente,
(b) 10 bis 70 Gew.-% des mindestens eines oberflächenaktiven Agens und
(c) 0 bis 50 Gew.-% des mindestens eines Lösungsmittels und/oder der Träger
substanz
umfasst.

3. Kosmetisches Produkt nach Anspruch 2, wobei der schweißabsorbierenden Komplex
(a) 20 bis 70 %, insbesondere etwa 30 bis 50 Gew.-% der mindestens einer wasserabsorbierenden Komponente,
(b) 20 bis 60 %, insbesondere etwa 30 bis 45 Gew.-% des mindestens eines oberflächenaktiven Agens und
(c) 0 bis 50 Gew.-% des mindestens eines Lösungsmittels und/oder der Trägersubstanz umfasst.

4. Kosmetisches Produkt nach einem der vorhergehenden Ansprüche **dadurch gekennzeichnet, dass** die zumindest eine wasserabsorbierende Komponente eine Wasserabsorptionskapazität von mindestens 20 %, insbesondere mindestens 50 % bezogen auf ihre Trockenmasse aufweist.

5. Kosmetisches Produkt nach einem der vorhergehenden Ansprüche **dadurch gekennzeichnet, dass** die mindestens eine wasserabsorbierende Komponente in Kontakt mit Wasser Partikel eines dreidimensionalen polymeren Netzwerks ausbildet

6. Kosmetisches Produkt nach Anspruch 5, **dadurch gekennzeichnet, dass** die mindestens eine wasserabsorbierende Komponente Partikel mit mittleren Partikelgrößen im Bereich von 0,1 bis 450 µm, insbesondere von 10 bis 200 µm, vorzugsweise von 10 bis 60 µm ausbildet.

7. Kosmetisches Produkt nach einem der vorhergehenden Ansprüche **dadurch gekennzeichnet, dass** die mindestens eine wasserabsorbierende Komponente in dem mindestens einen Lösungsmittel und/oder der Trägersubstanz im Wesentlichen unlöslich ist.

8. Kosmetisches Produkt nach einem der vorhergehenden Ansprüche **dadurch gekennzeichnet, dass** die mindestens eine wasserabsorbierende Komponente aus Gummis aus pflanzlicher oder mikrobieller Biosynthese gewählt ist, die im Wesentlichen eine Wiederholung von Monosaccharideinheiten umfassen und eine molekulare Masse von mindestens 100.000 g/mol aufweisen, insbesondere aus der Gruppe gewählt ist enthaltend Guar-Gummi, Guar-Derivate, Johannisbrotkernmehl, Scleroglucan, Tamarindenkernmehl, Dextrin-Gummi, Derivate und Mischungen von diesen.

9. Kosmetisches Produkt nach einem der vorhergehenden Ansprüche **dadurch gekennzeichnet, dass** die mindestens eine wasserabsorbierende Komponente aus Polysacchariden pflanzlichen Ursprungs gewählt ist, die im Wesentlichen eine Wiederholung von Monosaccharideinheiten von Hexosen und/oder Pentosen umfassen, insbesondere aus der Gruppe gewählt ist enthaltend natürliche Cellulosen, natürliche Fasern, Cellulose-Derivate, mikrokristalline Cellulose, Methylcellulosen, Methoxycellulosen, Hydroxyethylcellulosen, Hydroxypropylcellulosen, Hydroxypropylmethylcellulosen, Dextrine, Maltodextrine, Inulin, Inulin-Derivate, Stärke, Stärke-Derivate, Derivate und Mischungen von diesen.

10. Kosmetisches Produkt nach einem der vorhergehenden Ansprüche **dadurch gekennzeichnet, dass** die mindestens eine wasserabsorbierende Komponente aus chemisch modifizierten Polysacchariden gewählt ist, insbesondere aus der Gruppe enthaltend Cellulose-Derivate, Stärke-Derivate, Pektin-Derivate, Stärkepfropfcopolymere und Mischungen von diesen.

11. Kosmetisches Produkt nach einem der vorhergehenden Ansprüche **dadurch gekennzeichnet, dass** die mindestens eine wasserabsorbierende Komponente aus auf Polyacrylaten basierenden synthetischen Polymeren gewählt ist, die insbesondere Wiederholungen von Acrylsäure, Acrylamid, Methacrylsäure, Derivate oder Mischungen von diesen umfassen, insbesondere Polyacrylamide.

12. Kosmetisches Produkt nach einem der vorhergehenden Ansprüche **dadurch gekennzeichnet, dass** die mindestens eine wasserabsorbierende Komponente aus Kieselsäuren und ihren Derivaten und Modifikationen gewählt ist, insbesondere aus der Gruppe enthaltend Polykieselsäuren ((SiO₂)ₘ x nH₂O), Kieselsäureanhydrid (SiO₂), pyrogene Kieselsäure, hydratisierte Kieselsäure (SiO₂ x H₂O), Silicagel, und/oder Silikatester.

13. Kosmetisches Produkt nach einem der Ansprüche 1 bis 12, **dadurch gekennzeichnet, dass**, wenn das Produkt im Wesentlichen wasserfrei ist, das mindestens eine oberflächenaktive Agens eine Hydrophilie/Lipophilie-Balance (HLB) von mindestens 4,5 aufweist, insbesondere mindestens 7, vorzugsweise im Bereich von 12 bis 18, besonders bevorzugt von 14 bis 16.

14. Kosmetisches Produkt nach Anspruch 13, **dadurch gekennzeichnet, dass**, wenn das Produkt im Wesentlichen wasserfrei ist, der Komplex eine Mischung von mindestens zwei oberflächenaktiven Agenzien aufweist, die eine effektive gewichtete mittlere Hydrophilie/Lipophilie-Balance (HLB) von mindestens 4,5 aufweist, insbesondere mindestens 7, vorzugsweise im Bereich von 12 bis 18, besonders bevorzugt von 14 bis 16.

15. Kosmetisches Produkt nach einem der Ansprüche 1 bis 12, **dadurch gekennzeichnet, dass**, wenn das Produkt wasserhaltig ist, das mindestens eine oberflächenaktive Agens oder eine Mischung von mindestens zwei oberflächenaktiven Agenzien eine Hydrophilie/Lipophilie-Balance (HLB) im Bereich von 2 bis 13, insbesondere von 3 bis 11, vorzugsweise von 5 bis 10 aufweist.

16. Kosmetisches Produkt nach einem der vorhergehenden Ansprüche **dadurch gekennzeichnet, dass** das mindestens eine oberflächenaktive Agens eine nicht-ionische, kationische oder amphotere Verbindung oder ein Mischung von diesen ist, insbesondere gewählt ist aus der Gruppe enthaltend Fettalkohole, ethoxylierte Alkohole, ethoxylierte Triglyceride, ethoxylierte Öle, Monoglyceride, Carboxylsäureester von Alkyl- oder Alkenylglycolen, C1-C40-Fettsäureesfer von Polyolen, C1-C40-Fettsäureether von Polyolen, C1-C40- Fettsäureester von Alkyl- oder Alkenylglycolen, Polyglycerinester, Polyglycerinester von C1-C40-Fettsäuren, Kohlenwasserstoff-abgeleitete Ester, Zuckerester und - polyester, alkoxylierte Zuckerester und -polyester, ethoxylierte Carboxylsäureester von C1-C40-Fettsäuren, Sorbitan- oder Polysorbatester von Fettsäuren, ethoxylierte Sorbitanester von Fettsäuren, ethoxylierte Zuckerether von Fettsäuren, alkoxylierte Derivate von C1-C40-Fettsäureestern von C1-C40-Fettalkoholen, alkoxylierte Derivate von C1-C40-Fettsäureethem von C1-C40-Fettalkoholen, Polyethylenglycolether, Polyethylenglycolester, ethoxylierte Polysiloxane, Alkylglycoside, Alkanolamide, Aminoxide, Fettsäureamide, Alkylamidoalkylamine, Alkylamine, Alkylimidazoline, alkylsubstituierte Aminosäuren und Mischungen von diesen.

17. Kosmetisches Produkt nach einem der vorhergehenden Ansprüche **dadurch gekennzeichnet, dass** das mindestens eine Lösungsmittel und/oder die mindestens eine Trägersubstanz ausgewählt Ist aus der Gruppe enthaltend Glycole, Glycerin, polare und unpolare Öle, Kohlenwasserstoffe, Ether, Ester, mittel- und langkettige Alkohole, alkoxylierte Alkohole, polyhydrierte Alkohole, Polyole und Mischungen von diesen, insbesondere aus der Gruppe enthaltend Propylenglycol, Dipropylenglycol, Ethylenglycol, Glycerin, Diglycerin, Diacetin, Triacetin, Isopropylpalmitat, Isododecan, Isohexadecan, hydrogeniertes Polydecen, Triglyceride, Mineralöl und Mischungen von diesen.

18. Kosmetisches Produkt nach einem der vorhergehenden Ansprüche, **dadurch gekennzeichnet, dass** das Produkt ausgewählt ist aus der Gruppe enthaltend Antiperspirantien, Deodorants, jegliche adstringierende oder deodorierende Präparate, Stifte, Sprays, Aerosole, Cremes, Sönnenschutzmittel, Aftershaves, Lotionen, Grundierungscremes und Make-ups.

19. Kosmetisches Produkt nach einem der vorhergehenden Ansprüche, **dadurch gekennzeichnet, dass** der schweißabsorbierende Komplex einen Massenantell von 0,05 bis 99 Gew.-%, insbesondere 0,1 bis 10 Gew.-%, vorzugsweise 1 bis 6 Gew.-% im Produkt aufweist.

20. Kosmetisches Produkt nach einem der vorhergehenden Ansprüche, weiterhin umfassend Hilfsstoffe und/oder Wirkstoffe ausgewählt aus der Gruppe enthaltend Pigmente, Farbstoffe, Antioxidantien, Konservierungsmittel, andere feuchtigkeitsspeichernde Substanzen, Weichmacher, Duftstoffe, Stabilisatoren, Adstringentien, Zellturnover-Promotoren, Zellprofiferations-Stimulatoren, entzündungshemmende Agenzien, antimikrobielle Agenzien, Hormonregulatoren, Enzyminhibitoren, UV-Absorber, Sonnenschutzstoffe und Mischungen von diesen.

21. Kosmetisches Produkt nach einem der vorhergehenden Ansprüche, **dadurch gekennzeichnet, dass** das Produkt ein im Wesentlichen wasserfreies Antiperspirant ist, das 0,1 bis 10 Gew.-%, des schweißabsorbiereriden Komplexes in einer Antiperspirantbasisformulierung enthält

22. Kosmetisches Produkt nach Anspruch 21, wobei die Antiperspirantbasisformulierung 30 bis 70 %, insbesondere 50 bis 60 Gew.-% mindestens eines unpolaren Lösungsmittels; 5 bis 20 %, insbesondere etwa 10 Gew.-% eines Wachses; 10 bis 30 %, insbesondere etwa 20 Gew.-% mindestens eines Gelierungs- oder Verdickungsmittels und 10 bis 30 %, insbesondere etwa 20 Gew.-% mindestens eines Adstringenzes enthält.

23. Kosmetisches Produkt nach Anspruch 22, wobei das Lösungsmittel Silikonöl, das Gelierungsmittel Stearylalkohol und das Adstringent ein Aluminium- oder Zirkoniumsalz, insbesondere ein Aluminiumoxid- und/oder ein Zirkoniumoxidsalz umfasst

24. Verfahren zur Herstellung eines schweißabsorbierenden Komplexes für ein kosmetisches Produkt nach einem der Ansprüche 1 bis 23, wobei die mindestens eine wasserabsorbierende Komponente und das mindestens eine oberflächenaktive Agens unter Rühren und Wärmezufuhr miteinander vermischt werden, bis eine im Wesentlichenhomogene Mischung erhalten wird.

25. Verfahren nach Anspruch 24, wobei das mindestens eine Lösungsmittel und/oder die mindestens eine Trägersubstanz zu der Mischung aus der mindestens einen wasserabsorbierenden Komponente und dem mindestens einen oberflächenaktiven Agens unter Rühren und Wärmezufuhr zugesetzt wird, bis eine im Wesentlichen homogene Mischung erhalten wird.

26. Verfahren nach Anspruch 24 oder 25, wobei die Schritte Mischen der mindestens einen wasserabsorbierenden Komponente und des mindestens einen oberflächenaktiven Agens und/oder der Zugabe des mindestens einen Lösungsmittels und/oder der mindestens einen Trägersubstanz bei einer Temperatur zwischen 50 und 100°C, insbesondere zwischen 60 und 90 °C, vorzugsweise zwischen 70 und 80°Cdurchgeführt werden.

27. Verfahren zur Herstellung eines kosmetischen Produkts nach einem der Ansprüche 1 bis 23, umfassend die Schritte Herstellung eines schweißabsorbierenden Komplexes nach einem der Ansprüche 24 bis 26 und Mischen des schweißabsorbierenden Komplexes mit einer Basisformulierung.

28. Verfahren nach Anspruch 27, wobei die Basisformulierung eine, mindestens ein Adstringent enthaltende Antiperspirantformulierung ist, insbesondere eine im Wesentlichen wasserfreie Antiperspirantformulierung.

## Claims

1. Perspiration-absorbent cosmetic product comprising
i) a base formulation and
ii) a perspiration-absorbent complex, comprising
(a) 0.1 to 90% by weight of at least one water-absorbent component,
(b) 0.1 to 80% by weight of at least one surface-active agent and
(c) 0 to 50% by weight of at least one solvent
and/or at least one carrier substance,
wherein the perspiration-absorbent complex forms particles made of a three-dimensional, water-swellable network of the at least one water-absorbent component, which particles are at least partially coated with the at least one surface-active agent and are emulsified in the base formulation.

2. Cosmetic product according to Claim 1, where the perspiration-absorbent complex comprises
(a) 10 to 80% by weight of the at least one water-absorbent component,
(b) 10 to 70% by weight of the at least one surface-active agent and
(c) 0 to 50% by weight of the at least one solvent and/or the carrier substance.

3. Cosmetic product according to Claim 2, where the perspiration-absorbent complex comprises
(a) 20 to 70%, in particular about 30 to 50% by weight, of the at least one water-absorbent component,
(b) 20 to 60%, in particular about 30 to 45% by weight, of the at least one surface-active agent and
(c) 0 to 50% by weight of the at least one solvent and/or the carrier substance.

4. Cosmetic product according to one of the preceding claims, **characterized in that** the at least one water-absorbent component has a water absorption capacity of at least 20%, in particular at least 50%, based on its dry mass.

5. Cosmetic product according to one of the preceding claims, **characterized in that** the at least one water-absorbent component, upon contact with water, forms particles of a three-dimensional polymeric network.

6. Cosmetic product according to Claim 5, **characterized in that** the at least one water-absorbent component forms particles with average particle sizes in the range from 0.1 to 450 µm, in particular from 10 to 200 µm, preferably from 10 to 60 µm.

7. Cosmetic product according to one of the preceding claims, **characterized in that** the at least one water-absorbent component is essentially insoluble in the at least one solvent and/or the carrier substance.

8. Cosmetic product according to one of the preceding claims, **characterized in that** the at least one water-absorbent component is selected from gums from vegetable or microbial biosynthesis which essentially comprise a repetition of monosaccharide units and have a molecular mass of at least 100 000 g/mol, in particular is selected from the group comprising guar gum, guar derivatives, locust bean seed flour, scleroglucan, tamarind seed flour, dextrin gum, derivatives and mixtures of these.

9. Cosmetic product according to one of the preceding claims, **characterized in that** the at least one water-absorbent component is selected from polysaccharides of vegetable origin which essentially comprise a repetition of monosaccharide units from hexoses and/or pentoses, in particular is selected from the group comprising natural celluloses, natural fibres, cellulose derivatives, microcrystalline cellulose, methylcelluloses, methoxycelluloses, hydroxyethyl-celluloses, hydroxypropylcelluloses, hydroxypropyl-methylcelluloses, dextrins, maltodextrins, inulin, inulin derivatives, starch, starch derivatives, derivates and mixtures of these.

10. Cosmetic product according to one of the preceding claims, **characterized in that** the at least one water-absorbent component is selected from chemically modified polysaccharides, in particular from the group comprising cellulose derivatives, starch derivatives, pectin derivatives, starch graft copolymers and mixtures of these.

11. Cosmetic product according to one of the preceding claims, **characterized in that** the at least one water-absorbent component is selected from synthetic polymers based on polyacrylates which comprise in particular repetitions of acrylic acid, acrylamide, methacrylic acid, derivatives or mixtures of these, in particular polyacrylamides.

12. Cosmetic product according to one of the preceding claims, **characterized in that** the at least one water-absorbent component is selected from silicas and their derivatives and modifications, in particular from the group comprising polysilicic acids ((SiO₂)ₘ × nH₂O), silicic anhydride (SiO₂), fumed silica, hydrated silica (SiO₂ × nH₂O), silica gel, and/or silicate esters.

13. Cosmetic product according to one of Claims 1 to 12, **characterized in that** if the product is essentially anhydrous, the at least one surface-active agent has a hydrophilic-lipophilic balance (HLB) of at least 4.5, in particular at least 7, preferably in the range from 12 to 18, particularly preferably from 14 to 16.

14. Cosmetic product according to Claim 13, **characterized in that** if the product is essentially anhydrous, the complex has a mixture of at least two surface-active agents which has an effective weighted average hydrophilic-lipophilic balance (HLB) of at least 4.5, in particular at least 7, preferably in the range from 12 to 18, particularly preferably from 14 to 16.

15. Cosmetic product according to one of Claims 1 to 12, **characterized in that** if the product is hydrous, the at least one surface-active agent or a mixture of at least two surface-active agents has a hydrophilic-lipophilic balance (HLB) in the range from 2 to 13, in particular from 3 to 11, preferably from 5 to 10.

16. Cosmetic product according to one of the preceding claims, **characterized in that** the at least one surface-active agent is a nonionic, cationic or amphoteric compound or a mixture of these, in particular selected from the group comprising fatty alcohols, ethoxylated alcohols, ethoxylated triglycerides, ethoxylated oils, monoglycerides, carboxylic acid esters of alkyl or alkenyl glycols, C1-C40-fatty acid esters of polyols, C1-C40-fatty acid ethers of polyols, C1-C40-fatty acid esters of alkyl or alkenyl glycols, polyglyceryl esters, polyglyceryl esters of C1-C40-fatty acids, hydrocarbon-derived esters, sugar esters and polyesters, alkoxylated sugar esters and polyesters, ethoxylated carboxylic acid esters of C1-C40-fatty acids, sorbitan or polysorbate esters of fatty acids, ethoxylated sorbitan esters of fatty acids, ethoxylated sugar ethers of fatty acids, alkoxylated derivatives of C1-C40-fatty acid esters of C1-C40-fatty alcohols, alkoxylated derivatives of C1-C40-fatty acid ethers of C1-C4 0- fatty alcohols, polyethylene glycol ethers, polyethylene glycol esters, ethoxylated polysiloxanes, alkyl glycosides, alkanolamides, amine oxides, fatty acid amides, alkylamidoalkylamines, alkylamines, alkylimidazolines, alkyl-substituted amino acids and mixtures of these.

17. Cosmetic product according to one of the preceding claims, **characterized in that** the at least one solvent and/or the at least one carrier substance is selected from the group comprising glycols, glyceryl, polar and nonpolar oils, hydrocarbons, ethers, esters, medium- and long-chain alcohols, alkoxylated alcohols, polyhydrogenated alcohols, polyols and mixtures of these, in particular from the group comprising propylene glycol, dipropylene glycol, ethylene glycol, glyceryl, diglyceryl, diacetin, triacetin, isopropyl palmitate, isododecane, isohexadecane, hydrogenated polydecene, triglycerides, mineral oil and mixtures of these.

18. Cosmetic product according to one of the preceding claims, **characterized in that** the product is selected from the group comprising antiperspirants, deodorants, any astringent or deodorizing preparations, sticks, sprays, aerosols, creams, sunscreen compositions, aftershaves, lotions, foundation creams and make-ups.

19. Cosmetic product according to one of the preceding claims, **characterized in that** the perspiration-absorbent complex has a mass fraction of from 0.05 to 99% by weight, in particular 0.1 to 10% by weight, preferably 1 to 6% by weight, in the product.

20. Cosmetic product according to one of the preceding claims, further comprising auxiliaries and/or active ingredients selected from the group comprising pigments, dyes, antioxidants, preservatives, other moisture-storage substances, emollients, fragrances, stabilizers, astringents, cell turnover promoters, cell proliferation stimulators, anti-inflammatory agents, antimicrobial agents, hormone regulators, enzyme inhibitors, UV absorbers, sunscreen substances and mixtures of these.

21. Cosmetic product according to one of the preceding claims, **characterized in that** the product is an essentially anhydrous antiperspirant which comprises 0.1 to 10% by weight of the perspiration-absorbent complex in an antiperspirant base formulation.

22. Cosmetic product according to Claim 21, where the antiperspirant base formulation comprises 30 to 70%, in particular 50 to 60% by weight, of at least one nonpolar solvent; 5 to 20%, in particular about 10% by weight, of a wax; 10 to 30%, in particular about 20% by weight, of at least one gelling agent or thickener, and 10 to 30%, in particular about 20% by weight, of at least one astringent.

23. Cosmetic product according to Claim 22, where the solvent comprises silicone oil, the gelling agent comprises stearyl alcohol and the astringent comprises an aluminium or zirconium salt, in particular an aluminium oxide and/or a zirconium oxide salt.

24. Process for the preparation of a perspiration-absorbent complex for a cosmetic product according to one of Claims 1 to 23, where the at least one water-absorbent component and the at least one surface-active agent are mixed together with stirring and with the introduction of heat until an essentially homogeneous mixture is obtained.

25. Process according to Claim 24, where the at least one solvent and/or the at least one carrier substance is added to the mixture of the at least one water-absorbent component and the at least one surface-active agent with stirring and with the introduction of heat until an essentially homogeneous mixture is obtained.

26. Process according to Claim 24 or 25, where the steps of mixing the at least one water-absorbent component and the at least one surface-active agent and/or adding the at least one solvent and/or the at least one carrier substance are carried out at a temperature between 50 and 100°C, in particular between 60 and 90°C, preferably between 70 and 80°C.

27. Process for the preparation of a cosmetic product according to one of Claims 1 to 23, comprising the steps of preparation of a perspiration-absorbent complex according to one of Claims 24 to 26 and mixing of the perspiration-absorbent complex with a base formulation.

28. Process according to Claim 27, where the base formulation is an antiperspirant formulation comprising at least one astringent, in particular an essentially anhydrous antiperspirant formulation.

## Revendications

1. Produit cosmétique absorbant la sueur, contenant
i) une formulation de base, et
ii) un complexe absorbant la sueur, comprenant
(a) 0,1 à 90 % en poids d'au moins un composant absorbant l'eau,
(b) 0,1 à 80 % en poids d'au moins un agent tensioactif, et
(c) 0 à 50 % en poids d'au moins un solvant
et/ou d'au moins une substance porteuse, dans lequel le complexe absorbant la sueur forme des particules d'un réseau tridimensionnel, capable de se développer dans l'eau, dudit au moins un composant absorbant l'eau, qui sont enrobées au moins partiellement avec ledit au moins un agent tensioactif et sont émulsionnées dans la formulation de base.

2. Produit cosmétique selon la revendication 1, dans lequel le complexe absorbant la sueur comprend
(a) 10 à 80 % en poids dudit au moins un composant absorbant l'eau,
(b) 10 à 70 % en poids dudit au moins un agent tensioactif, et
(c) 0 à 50 % en poids dudit au moins un solvant et/ou de ladite substance porteuse.

3. Produit cosmétique selon la revendication 2, dans lequel le complexe absorbant la sueur comprend
(a) 20 à 70 % en poids, en particulier environ 30 à 50 % en poids dudit au moins un composant absorbant l'eau,
(b) 20 à 60 % en poids, en particulier environ 30 à 45 % en poids dudit au moins un agent tensioactif, et
(c) 0 à 50 % en poids dudit au moins un solvant et/ou de ladite substance porteuse.

4. Produit cosmétique selon l'une quelconque des revendications précédentes, **caractérisé en ce que** ledit au moins un composant absorbant l'eau présente une capacité d'absorption d'eau d'au moins 20 %, en particulier d'au moins 50 %, rapportée à sa masse à sec.

5. Produit cosmétique selon l'une quelconque des revendications précédentes, **caractérisé en ce que** ledit au moins un composant absorbant l'eau forme, au contact de l'eau, des particules d'un réseau polymère tridimensionnel.

6. Produit cosmétique selon la revendication 5, **caractérisé en ce que** ledit au moins un composant absorbant l'eau forme des particules d'une taille moyenne de particules dans la plage de 0,1 à 450 µm, en particulier de 10 à 200 µm, de préférence de 10 à 60 µm.

7. Produit cosmétique selon l'une quelconque des revendications précédentes, **caractérisé en ce que** ledit au moins un composant absorbant l'eau est essentiellement insoluble dans ledit au moins un solvant et/ou la substance porteuse.

8. Produit cosmétique selon l'une quelconque des revendications précédentes, **caractérisé en ce que** ledit au moins un composant absorbant l'eau est choisi parmi des gommes provenant de la biosynthèse végétale ou microbienne, qui comprennent essentiellement une répétition d'unités de monosaccharides et présentent une masse moléculaire d'au moins 100 000 g/mole, en particulier est choisi dans le groupe contenant de la gomme de guar, des dérivés de guar, de la farine de caroube, du scléroglucane, de la farine de tamarinier, de la gomme dextrine, des dérivés et des mélanges de ceux-ci.

9. Produit cosmétique selon l'une quelconque des revendications précédentes, **caractérisé en ce que** ledit au moins un composant absorbant l'eau est choisi parmi des polysaccharides d'origine végétale, qui comprennent essentiellement une répétition d'unités de monosaccharides de hexoses et/ou de pentoses, en particulier est choisi dans le groupe contenant des celluloses naturelles, des fibres naturelles, des dérivés de la cellulose, la cellulose microcristalline, des méthylcelluloses, des méthoxycelluloses, des hydroxyéthylcelluloses, des hydroxypropylcelluloses, des hydroxypropylméthylcelluloses, des dextrines, des maltodextrines, l'inuline, des dérivés de l'inuline, l'amidon, des dérivés de l'amidon, des dérivés et des mélanges de ceux-ci.

10. Produit cosmétique selon l'une quelconque des revendications précédentes, **caractérisé en ce que** ledit au moins un composant absorbant l'eau est choisi parmi les polysaccharides chimiquement modifiés, en particulier dans le groupe contenant des dérivés de la cellulose, des dérivés de l'amidon, des dérivés de la pectine, des copolymères greffés à l'amidon, et des mélanges de ceux-ci.

11. Produit cosmétique selon l'une quelconque des revendications précédentes, **caractérisé en ce que** ledit au moins un composant absorbant l'eau est choisi parmi des polymères synthétiques à base de polyacrylates, qui comprennent en particulier des répétitions de l'acide acrylique, acrylamide, acide méthacrylique, des dérivés ou des mélanges de ceux-ci, en particulier des polyacrylamides.

12. Produit cosmétique selon l'une quelconque des revendications précédentes, **caractérisé en ce que** ledit au moins un composant absorbant l'eau est choisi parmi les acides siliciques et leurs dérivés et modificateurs, en particulier dans le groupe contenant les acides polysiliciques ((SiO₂)ₘ x nH₂O) , l'anhydride silicique (SiO₂), l'acide silicique pyrogénique, l'acide silicique hydraté (SiO₂ x nH₂O), le gel de silice, et/ou des esters de silicates.

13. Produit cosmétique selon l'une quelconque des revendications 1 à 12, **caractérisé en ce que**, lorsque le produit est essentiellement exempt d'eau, ledit au moins un agent tensioactif présente un bilan hydrophilie/lipophilie (HLB) d'au moins 4,5, en particulier d'au moins 7, de préférence dans la plage de 12 à 18, et de préférence encore de 14 à 16.

14. Produit cosmétique selon la revendication 13, **caractérisé en ce que**, lorsque le produit est essentiellement exempt d'eau, le complexe présente un mélange d'au moins deux agents tensioactifs, qui présente un bilan hydrophilie/lipophilie (HLB) moyen pondéré effectif d'au moins 4,5, en particulier d'au moins 7, de préférence dans la plage de 12 à 18, et de préférence encore de 14 à 16.

15. Produit cosmétique selon l'une quelconque des revendications 1 à 12, **caractérisé en ce que**, lorsque le produit contient de l'eau, ledit au moins un agent tensioactif ou un mélange d'au moins deux agents tensioactifs présente un bilan hydrophilie/lipophilie (HLB) dans la plage de 2 à 13, en particulier de 3 à 11, de préférence de 5 à 10.

16. Produit cosmétique selon l'une quelconque des revendications précédentes, **caractérisé en ce que** ledit au moins un agent tensioactif est un composé non ionique, cationique ou amphotère ou un mélange de ceux-ci, en particulier il est choisi dans le groupe contenant les alcools gras, les alcools éthoxylés, les triglycérides éthoxylés, les huiles éthoxylées, les monoglycérides, les esters d'acides carboxyliques d'alkyl- ou d'alcénylglycols, les esters d'acides gras C₁-C₄₀ de polyols, les éthers d'acides gras C₁-C₄₀ de polyols, les esters d'acides gras C₁-C₄₀ d'alkyl- ou d'alcénylglycols, les esters de polyglycérol, les esters de polyglycérol d'acides gras C₁-C₄₀, les esters dérivés d'hydrocarbures, les esters et polyesters de sucre, les esters et polyesters de sucre alcoxylés, les esters d'acide carboxylique éthoxylés d'acides gras C₁-C₄₀, les esters de sorbitane et de polysorbate d'acides gras, les esters de sorbitane éthoxylés d'acides gras, les éthers de sucre éthoxylés d'acides gras, les dérivés alcoxylés d'esters d'acides gras C₁-C₄₀ d'alcools gras C₁-C₄₀, les dérivés alcoxylés d'éthers d'acides gras C₁-C₄₀ d'alcools gras C₁-C₄₀, l'éther de polyéthylène glycol, l'ester de polyéthylène glycol, les polysiloxanes éthoxylés, les alkyl glucosides, les alcanolamides, les aminoxydes, les amides d'acides gras, les alkylamidoalkylamines, les alkylamines, les alkylimidazolines, les acides aminés substitués par alkyle et des mélanges de ceux-ci.

17. Produit cosmétique selon l'une quelconque des revendications précédentes, **caractérisé en ce que** ledit au moins un solvant et/ou ladite au moins une substance porteuse est sélectionné(e) dans le groupe contenant les glycols, le glycérol, les huiles polaires et non polaires, les hydrocarbures, l'éther, l'ester, les alcools à chaîne moyenne et longue, les alcools alcoxylés, les alcools polyhydriques, les polyols et des mélanges de ceux-ci, en particulier dans le groupe contenant le propylène glycol, le dipropylène glycol, l'éthylène glycol, le glycérol, le diglycérol, le diacétine, le triacétine, le palmitate d'isopropyle, l'isododécane, l'isohexadécane, le polydécène hydrogéné, les triglycérides, l'huile minérale et des mélanges de ceux-ci.

18. Produit cosmétique selon l'une quelconque des revendications précédentes, **caractérisé en ce que** le produit est sélectionné dans le groupe contenant les antiperspirants, les désodorisants, les préparations astringentes ou désodorisantes, les crayons, les sprays, les aérosols, les crèmes, les produits de protection solaire, les après-rasage, les lotions, les crèmes de fond de teint et les maquillages.

19. Produit cosmétique selon l'une quelconque des revendications précédentes, **caractérisé en ce que** le complexe absorbant la sueur présente une proportion massique de 0,05 à 99 % en poids, en particulier 0,1 à 10 % en poids, de préférence 1 à 6 % en poids dans le produit.

20. Produit cosmétique selon l'une quelconque des revendications précédentes, comprenant en outre des substances auxiliaires et/ou des substances actives sélectionnées dans le groupe contenant des pigments, des colorants, des antioxydants, des agents de conservation, d'autres substances accumulant l'humidité, des assouplissants, des parfums, des stabilisants, des astringents, des promoteurs de rénovation des cellules, des stimulants de prolifération des cellules, des agents combattant l'inflammation, des agents antimicrobiens, des régulateurs d'hormones, des inhibiteurs d'enzymes, des absorbeurs d'UV, des substances de protection solaire et des mélanges de ceux-ci.

21. Produit cosmétique selon l'une quelconque des revendications précédentes, **caractérisé en ce que** le produit est un antiperspirant essentiellement exempt d'eau, qui contient 0,1 à 10 % en poids du complexe absorbant la sueur dans une formulation de base antiperspirante.

22. Produit cosmétique selon la revendication 21, dans lequel la formulation de base antiperspirante contient 30 à 70 % en poids, en particulier 50 à 60 % en poids d'au moins un solvant non polaire; 5 à 20 % en poids, en particulier environ 10 % en poids d'une cire; 10 à 30 % en poids, en particulier environ 20 % en poids d'au moins un agent de gélification ou d'épaississement et 10 à 30 % en poids, en particulier environ 20 % en poids d'au moins un astringent.

23. Produit cosmétique selon la revendication 22, dans lequel le solvant comprend une huile silicone, l'agent de gélification comprend de l'alcool stéarylique et l'astringent comprend un sel d'aluminium ou de zirconium, en particulier un sel d'oxyde d'aluminium et/ou d'oxyde de zirconium.

24. Procédé pour la fabrication d'un complexe absorbant la sueur pour un produit cosmétique selon l'une quelconque des revendications 1 à 23, dans lequel on mélange l'un à l'autre ledit au moins un composant absorbant l'eau et ledit au moins un agent tensioactif avec agitation et apport de chaleur, jusqu'à ce que l'on obtienne un mélange essentiellement homogène.

25. Procédé selon la revendication 24, dans lequel on ajoute ledit au moins un solvant et/ou ladite au moins une substance porteuse au mélange dudit au moins un composant absorbant l'eau et dudit au moins un agent tensioactif avec agitation et apport de chaleur, jusqu'à ce que l'on obtienne un mélange essentiellement homogène.

26. Procédé selon la revendication 24 ou 25, dans lequel on exécute les étapes de mélange dudit au moins un composant absorbant l'au et dudit au moins un agent tensioactif et/ou d'addition dudit au moins un solvant et/ou de ladite au moins une substance porteuse à une température comprise entre 50 et 100°C, en particulier entre 60 et 90°C, de préférence entre 70 et 80°C.

27. Procédé pour la fabrication d'un produit cosmétique selon l'une quelconque des revendications 1 à 23, comprenant les étapes de fabrication d'un complexe absorbant la sueur selon l'une quelconque des revendications 24 à 26 et de mélange du complexe absorbant la sueur avec une formulation de base.

28. Procédé selon la revendication 27, dans lequel la formulation de base est une formulation antiperspirante, contenant au moins un astringent, en particulier une formulation antiperspirante essentiellement exempte d'eau.
